# EUROPÄISCHE PATENTANMELDUNG

(11) **EP 2 353 626 A1**
(43) Veröffentlichungstag der Anmeldung: **10.08.2011**
(21) Anmeldenummer: 10075043.9
(22) Anmeldetag: 27.01.2010
(51) Int. Cl.: A61M 1/10, F04D 33/00

(54) **Fördereinrichtung für ein Fluid**

(71) Anmelder: ECP Entwicklungsgesellschaft mbH, 12247 Berlin (DE)
(72) Erfinder: Liebing, Reiner, 14469 Potsdam (DE)
(74) Vertreter: Pfenning, Meinig & Partner GbR

(57) **Zusammenfassung**

Die Erfindung bezieht sich auf eine Fördereinrichtung zur Förderung eines Fluids in einer Förderrichtung (9) mit einem oder mehreren Antriebskörpern (1,1',1",1"',11,11',11",11"'), die mittels eines Antriebssystems (15,15') oszillierend quer zur Förderrichtung antreibbar sind. Durch entsprechende translatorische oder teils schwenkende Bewegung der Antriebskörper wird nach Art des aus der Biologie (z. B. Aerodynamik und Hydrodynamik) bekannten Flossenprinzips eine Beschleunigung des Fluids erreicht.

## Beschreibung

Die Erfindung liegt auf dem Gebiet des Maschinenbaus und befasst sich mit Fördereinrichtungen für Fluide, insbesondere für Flüssigkeiten.

Solche Fördereinrichtungen sind in Form verschiedener Arten von Pumpen bereits in vielfältigsten Ausführungsformen bekannt geworden. Insbesondere interessant sind an dieser Stelle Pumpen, die in derartigen Bauformen hergestellt werden können, dass sie auch für sensiblere, insbesondere Fluide mit Makromolekülen eingesetzt werden können. Eine spezielle Gruppe unter derartigen Pumpen stellen die für medizinische Anwendungszwecke verwendbaren und in kleinen Bauformen herstellbaren Fluidpumpen dar. Solche Pumpen können in Mikrobauformen beispielsweise auch zur Förderung von körpereigenen oder bioverträglichen Flüssigkeiten eingesetzt werden, beispielsweise als Herzpumpen zur Förderung von Blut.

Bei der Förderung solcher sensibler Flüssigkeiten wie Blut, die beispielsweise große und empfindliche Moleküle aufweisen, die biologische Funktionen erfüllen und deshalb auch auf mikroskopischer Ebene nicht beschädigt werden dürfen, ist darauf zu achten, dass die mechanische Einwirkung auf die Flüssigkeit durch Druckmaxima, Scherkräfte und Beschleunigungen möglichst begrenzt wird.

Insbesondere zur Förderung von Blut sind in diesem Zusammenhang beispielsweise Axialpumpen bekannt geworden, die einen um eine Längsachse rotierenden Rotor mit Förderschaufeln aufweisen, der das Blut in Axialrichtung kontinuierlich fördert.

Da ein spezielles Problem für den Einsatz solcher Pumpen im Körperinneren darin besteht, diesen einerseits eine ausreichende Förderkapazität zu verleihen, andererseits aber die Baugröße so zu gestalten, dass sie durch ein Blutgefäß eingeführt werden können, liegen einige der Herausforderungen für solche Pumpen darin, sie konstruktiv so zu gestalten, dass sie radial komprimierbar und zum Betrieb im Körper wieder expandierbar sind.

Ein derartiger komprimierbarer Rotor ist beispielsweise aus der US 6 860 713 bekannt. Ein anderer Rotor ist aus der US 7 393 181 B2 bekannt. Bei den bekannten Lösungen sind die Rotoren entweder wegen der Elastizität und Verformbarkeit des Materials oder aufgrund von mechanisch beweglichen Konstruktionen komprimierbar und expandierbar.

Es ist dabei unvermeidlich, dass ein gewisser konstruktiver Aufwand getrieben wird, um die Komprimierbarkeit einer solchen Pumpe trotz entsprechender Zuverlässigkeit und Förderkapazität sicherzustellen. Zudem muss sichergestellt werden, dass nicht durch eine zu hohe Rotationsgeschwindigkeit des Rotors oder ungünstige geometrische Formen der Förderschaufeln zu große Scherkräfte entstehen, die sensible Flüssigkeiten schädigen können. Zudem ist darauf zu achten, dass Druckunterschiede innerhalb der Geometrie einer solchen Fördereinrichtung einerseits und im zeitlichen Ablauf andererseits in engen Grenzen gehalten werden.

Bei diesen Randbedingungen und vor dem Hintergrund des Standes der Technik liegt der vorliegenden Erfindung die Aufgabe zugrunde, eine Fördereinrichtung zu schaffen, die mit konstruktiv einfachen Mitteln herstellbar ist und zuverlässig und schonend die Förderung eines Fluids ermöglicht.

Die Aufgabe wird gemäß der Erfindung mit den Merkmalen des Patentanspruchs 1 gelöst.

Die erfindungsgemäße Fördereinrichtung, die dazu dient, ein Fluid in einer Förderrichtung zu bewegen, weist zu diesem Zweck einen mittels eines Antriebssystems antreibbaren Antriebskörper auf, der quer zur Förderrichtung oszillierend antreibbar ist.

Der Antriebskörper ist dazu in einem Kanal oder einem Raum angeordnet, in dem das Fluid in einer vorgegebenen Förderrichtung gefördert werden soll.

Bekannte Fördermechanismen wie beispielsweise Kreiselpumpen oder die oben genannten Axialpumpen bedienen sich rotierender Förderelemente zur Bewegung bzw. Beschleunigung eines Fluids. Die ebenfalls bekannten Kolbenpumpen weisen jeweils wenigstens einen Kolben auf, der im Wesentlichen translatorisch bewegbar ist und bei seiner Bewegung das Medium in seiner Bewegungsrichtung fördert.

Im Gegensatz dazu wird gemäß der vorliegenden Erfindung der Antriebskörper quer zur Förderrichtung nach Art einer Fischflosse bewegt, die in der Natur regelmäßig zur Erzeugung einer Relativbewegung zwischen der Flosse und einem Fluid eingesetzt wird. Bei der vorliegenden Erfindung steht dabei das flossenartige Element, der Antriebskörper in der Förderrichtung im Wesentlichen fest, so dass die Relativbewegung in einer Förderbewegung des Fluids resultiert.

Die Bewegung des Antriebskörpers quer zur Förderrichtung bedeutet dabei beispielsweise, dass wenigstens ein Teil des Antriebskörpers translatorisch oder entlang einer wenig gekrümmten Bahn im Wesentlichen senkrecht zur Förderrichtung und/oder verbunden mit einer Schwenkbewegung um eine Achse bewegt wird, die im Wesentlichen senkrecht zur Förderrichtung steht. Dabei sollte die Abweichung der Verlaufsrichtung der Achse zur Senkrechten der Förderrichtung maximal 45° betragen. Damit sollen die aus der Bionik bekannten Bewegungsmuster von flossenartigen Körpern bei Fischen und anderen Lebewesen nachgebildet werden.

Die entsprechenden Antriebskörper können in ihrer Form und Größe an den zur Verfügung stehenden Raum angepasst werden. Die Relativbewegung des Antriebskörpers bzw. verschiedener seiner Teile gegenüber dem anzutreibenden Fluid kann bezüglich der Geschwindigkeit in einem Bereich gehalten werden, der das Entstehen von unzulässigen Scherkräften verhindert. Dabei ist die Relativgeschwindigkeit auf die Viskosität des zu fördernden Mediums und entsprechend ggf. vorliegende Kompressibilitäten abzustimmen. Das beschriebene Förderprinzip ist bei im Wesentlichen inkompressiblen und leicht flüssigen Medien wie beispielsweise Blut besonders effizient einsetzbar. Auch entsprechende Antriebsbewegungen lassen sich zu einem oszillierend zu bewegenden Antriebskörper gut übertragen. Es muss nicht notwendig eine drehbare Lagerung eines Rotors vorgesehen werden.

Da durch die oszillatorische Bewegung des Antriebskörpers eine gewisse Periodizität von Druckschwankungen zu erwarten ist, bei denen eine zeitweise Umkehr der Strömungsrichtung nicht immer ausgeschlossen werden kann, kann vorteilhaft auch die Anordnung eines Steuerventils für die zu erzeugende Strömung im Förderkanal bzw. in dem Raum, in dem sich der Antriebskörper befindet, in Betracht gezogen werden. Dabei kann das Ventil entweder durch eine intelligente Steuerung synchron zur Bewegung des Antriebskörpers gesteuert oder als selbsttätig wirkendes Rückschlagventil ausgeführt werden.

Vorteilhaft wird die Förderfläche oder eine Förderfläche des Antriebskörpers derart ausgerichtet, dass bei einer Bewegung des Antriebskörpers auf das Fluid eine Teilkraft in der Förderrichtung wirkt. Hierzu sind die Bewegungsrichtung des Antriebskörpers und die Verlaufsrichtung der Flächen des Antriebskörpers, an denen eine Druckerhöhung entsteht, entsprechend aufeinander abzustimmen.

Es können in diesem Zusammenhang auch wenigstens zwei Förderflächen, beispielsweise an einem einzigen Antriebskörper, vorgesehen sein, die derart ausgerichtet sind, dass sie jeweils in wenigstens einer der Bewegungsrichtungen des Antriebskörpers eine Förderung des Fluids bewirken. Damit wird eine Förderung des Fluids in beiden oder mehreren Antriebsbewegungsrichtungen möglich.

Es kann außerdem vorteilhaft vorgesehen sein, dass wenigstens ein Antriebskörper sich in Förderrichtung in dem parallel zu seiner Bewegungsebene liegenden Querschnitt verjüngt.

Der Antriebskörper kann beispielsweise flossenartig als keilförmiger Körper ausgebildet sein, dessen verdicktes Ende bezüglich der zu erzeugenden Strömung stromaufwärts und dessen verjüngtes Ende stromabwärts angeordnet ist. Das verdjüngte Ende kann in Form einer Schneide spitz zulaufen, wobei die Schneide senkrecht zur Antriebsrichtung des Antriebskörpers verlaufen kann. Der Antriebskörper kann auch in der Verlaufsrichtung der Schneide zu seinem verjüngten Ende hin verbreitert sein.

Die Förderflächen zu beiden Seiten eines derart keilförmigen Flossenkörpers können entweder eben oder konvex oder konkav, in Richtung senkrecht zur Antriebsrichtung des Antriebskörpers gesehen, ausgebildet sein.

Der Antriebskörper kann in einer Ausführungsart der Erfindung steif ausgebildet sein. In diesem Fall kann der Antriebskörper um eine Achse, die im Bereich seines verdickten Endes liegt, schwenkbar sein. Zusätzlich kann eine überlagerte translatorische Bewegung des verdickten Endes, beispielsweise gerade oder entlang einer Kulissenbahn, vorgegeben sein. Der translatorische Bewegungsanteil findet dabei in derselben Ebene statt wie die Schwenkbewegung. Alternativ dazu kann auch vorgesehen sein, dass der Antriebskörper derart elastisch ausgebildet ist, dass er durch den Fluidgegendruck im Betrieb in seinem Endbereich um wenigstens 5°, insbesondere auch wenigstens 20° gegenüber dem unverformten Zustand biegbar ist.

Der Antrieb kann in diesem Fall ebenso ausgebildet sein wie bei einem steifen Antriebskörper, jedoch wird durch die Elastizität und Verformbarkeit des Antriebskörpers an sich schon die Ausrichtung der Förderflächen relativ zum zu fördernden Fluid in der jeweiligen Phase der Antriebsbewegung optimiert und damit die Effizienz des Antriebs erhöht.

Ein derartiger Antriebskörper, gleich ob steif oder elastisch ausgebildet, kann in dem senkrecht zur Ebene der Antriebsbewegung betrachteten Querschnitt entweder symmetrisch keilförmig, mit ebenen, konkaven oder konvexen Förderflächen ausgebildet sein, oder es kann auch eine in dem genannten Querschnitt asymmetrische Form, beispielsweise mit Elementen eines Tragflügels vorgesehen sein, um zusätzliche Strömungseffekte zu nutzen. Ein derartiges Tragflügelprofil sieht beispielsweise auf einer Seite des Antriebskörpers eine konkave Form, auf der gegenüberliegenden Seite eine konvexe oder gerade Form der Förderfläche vor.

Bei Verwendung einer derart asymmetrischen Gestaltung eines Antriebskörpers kann ein weiterer Antriebskörper zusätzlich vorgesehen sein, der spiegelbildlich zu dem ersten Antriebskörper geformt und angeordnet ist und synchronisiert mit diesem gleichsinnig oder gegensinnig bewegbar ist.

Zur Erhöhung der Effizienz des Antriebs kann zudem vorgesehen sein, dass der Antriebskörper, insbesondere im Bereich einer Förderfläche, optimierte Oberflächenstrukturen aufweist.

In einer vorteilhaften Ausführungsform der Erfindung kann außerdem vorgesehen sein, dass der Antriebskörper wenigstens einen Hohlraum aufweist. Das Vorsehen eines Hohlraums verringert die Masse des Antriebskörpers und damit die aufzubringende Energie für seine Beschleunigung. Zudem kann der Antriebskörper wenigstens teilweise aufpumpbar gestaltet sein, so dass seine äußeren Abmaße im nicht aufgepumpten Zustand geringer sein können als im aufgepumpten Zustand. Ein derartiger Antriebskörper kann dann leichter im nicht aufgepumpten Zustand an einen Einsatzort gebracht und dort auf die Betriebsmaße aufgepumpt werden. Dies ist insbesondere dann vorteilhaft, wenn die Fördereinrichtung in kleinsten Abmaßen hergestellt und innerhalb von Blutgefäßen bewegt werden soll.

Der Antriebskörper kann vorteilhaft zudem aus einem Schaumstoff, insbesondere aus Polyurethan bestehen. Damit kann der Antriebskörper elastisch verformbar und sehr leicht hergestellt werden.

Durch Vorsehen eines entsprechenden Antriebssystems kann bei der erfindungsgemäßen Fördereinrichtung vorgesehen sein, dass der Antriebskörper mittels eines hydraulischen oder pneumatischen Einrichtung, insbesondere eines Ballonkörpers, jedoch auch mittels einer elektrischen und/oder magnetischen Einrichtung, antreibbar ist.

Obwohl einer oder mehrere Antriebskörper gemäß der Erfindung mittels Hebeln oder ähnlicher mechanischer Einrichtungen einfach bewegbar sind, kann durch eine hydraulische oder pneumatische Antriebseinrichtung die Antriebsbewegung besonders einfach zu der Fördereinrichtung geleitet werden. Entsprechende pneumatische oder hydraulische Leitungen können beispielsweise in Form eines Hohlkatheters oder auch innerhalb eines Hohlkatheters, an dessen distalem Ende die Fördereinrichtung vorgesehen ist, verlegt werden und im Bereich der Fördereinrichtung entweder direkt auf einen Kolben, Faltenbalg oder ballonartigen Antriebskörper wirken oder dort in eine Hebelbewegung umgesetzt werden.

Mögliche Antriebsbewegungen des oder der Antriebskörper sehen dabei vor, dass wenigstens ein Antriebskörper oszillierend um eine quer zur Förderrichtung verlaufende Achse schwenkbar ist und/oder dass einer oder mehrere Antriebskörper oszillierend um eine in Förderrichtung, insbesondere außerhalb der Förderkörper, verlaufende Achse schwenkbar sind.

Besonders an einer derartigen oszillatorischen Bewegung ist, dass die Schwenkbewegung einen relativen geringen Hub aufweist, so dass in keinem Fall eine volle Drehung des Antriebskörpers erfolgt.

Bei der Drehung um eine in Förderrichtung verlaufende Achse kann jedoch auch eine Drehung um größere Winkel vorgesehen sein.

Zur Verringerung von unerwünschten Druckausgleichsvorgängen an den Antriebskörpern können an diesen zwischen deren Förderflächen Sperrkörper angeordnet sein. Diese sollten flexibel sein, können dabei biegeschlaff oder steif, aber biegsam ausgebildet sein. Die Sperrkörper können auch jeweils zwei Sperrkörper miteinander oder einen Sperrkörper mit einer Gehäusewand verbinden.

Das beschriebene flossenartige Antriebsprinzip für Fluide ist im Zusammenhang mit der Förderung von Flüssigkeiten neu und erlaubt damit die Realisierung von Fördercharakteristiken, die mit den bereits bekannten Fördereinrichtungen nicht erreichbar sind.

Im Folgenden wird die Erfindung anhand eines Ausführungsbeispiels in einer Zeichnung gezeigt und anschließend beschrieben.

Dabei zeigt
- Fig. 1: im Querschnitt einen Antriebskörper in drei Stellungen,
- Fig. 2: in einem Längsschnitt ein Fördersystem für Fluide mit zwei Antriebskörpern,
- Fig. 3: ein Fördersystem mit zwei Antriebskörpern in einer dreidimensionalen Ansicht,
- Fig. 4: zwei Antriebskörper in einer ersten Stellung mit einem Antriebssystem,
- Fig. 5: die Antriebskörper aus Fig. 4 in einer zweiten Stellung,
- Fig. 6: die Antriebskörper aus den Fign. 4 und 5 in einer dritten Stellung,
- Fig. 7: ein Antriebssystem in dreidimensionaler Darstellung mit einem im Querschnitt vier-

- Fig. 8: eckigen Förderraum, zwei Antriebskörper, die oszillierend um eine in Förderrichtung verlaufende Achse gedreht werden,
- Fig. 9: in dreidimensionaler Ansicht ein Antriebssystem mit zwei teilzylindrisch ausgebildeten Antriebskörpern,
- Fig. 10: einen Schnitt durch das Antriebssystem aus Fig. 9,
- Fig. 11: eine Ausführungsform wie Fig. 3 mit zusätzlichen Sperrkörpern,
- Fig. 12: eine Ausführungsform ähnlich der aus Fig. 7 mit Sperrkörpern,
- Fig. 13: eine Darstellung von zwei Antriebskörpern, die mittels Sperrkörpern verbunden sind,
- Fig. 14: die Ausführungsform aus Fig. 13 in einer Frontansicht,
- Fig. 15: eine Ansicht der Ausführungsform aus Fig. 13, wobei die Einwirkung einer Antriebskraft auf die Sperrkörper angedeutet ist,
- Fig. 16: eine Anordnung, bei der die Sperrkörper eine steife, aber biegsame Ringstreifenform aufweisen,
- Fig. 17: einen Antriebskörper mit fin-rays im neutralen Zustand sowie

- Fig. 18: einen Antriebskörper wie in Fig. 17 im belasteten Zustand.

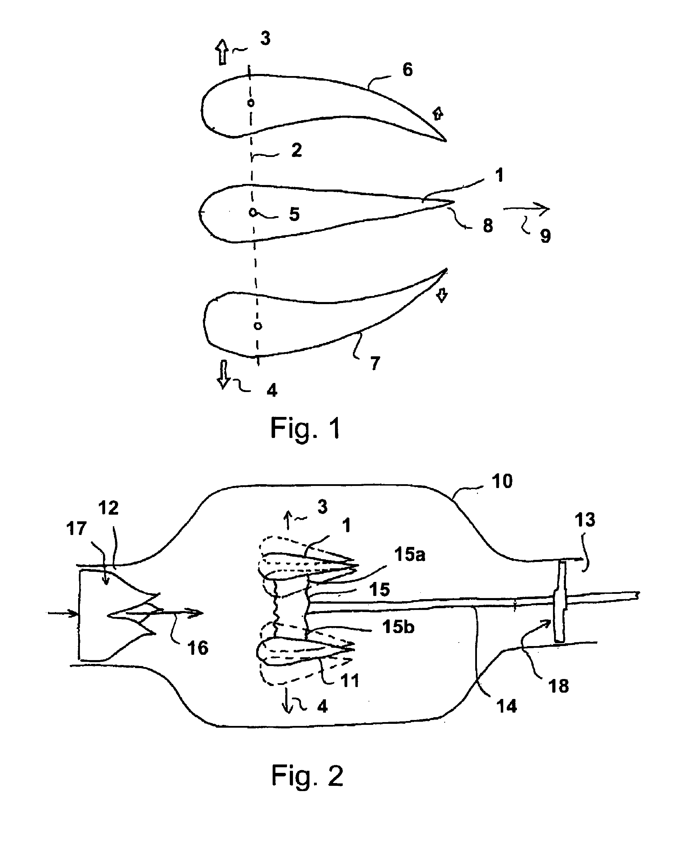

Fig. 1 zeigt im mittleren Teil einen Antriebskörper 1 im Profil, der im Wesentlichen eine Keilform aufweist, die der Form einer in der Biologie auftretenden Flosse nachgebildet ist. Der Antriebskörper 1 erstreckt sich senkrecht zur Zeichenebene mit gleichbleibendem Profil, kann sich allerdings zu seinem verjüngten Ende hin auch senkrecht zur Zeichenebene verbreitern.

Der Antriebskörper 1 kann entlang der gestrichelten Linie 2 in den durch die Pfeile 3, 4 angedeuteten Richtungen oszillierend bewegt werden. Dabei ist der Bereich um den Angriffspunkt der Antriebskraft als Kreis dargestellt und mit 5 bezeichnet. An diesem Punkt greift die Antriebskraft derart an, dass der Antriebskörper im Wesentlichen translatorisch entlang der Linie 2 bewegt wird und in einer ersten Variante somit nicht schwenkbar ist, um einem wirkenden Fluidgegendruck auszuweichen.

Es ergibt sich dann im Betrieb beispielsweise bei Bewegung des Antriebskörpers innerhalb einer Flüssigkeit auf der Seite der jeweils wirkenden Förderfläche 6, 7 ein Fluidgegendruck, der zu einer Deformation des antriebsfernen Endes 8 des Antriebskörpers 1, d. h. des verjüngten Endes, führt, wenn dieser Antriebskörper wie in dem gezeigten Beispiel elastisch ausgebildet ist. Durch diesen Effekt ergibt sich ein besonders effizienter Vortrieb des angetriebenen Fluids in der Förderrichtung 9.

Alternativ kann der Antrieb des Antriebskörpers 1 auch so ausgestaltet sein, dass dieser nicht streng translatorisch im Sinne der Richtungen 3, 4, sondern in einer überlagerten translatorischen Bewegung und Schwenkbewegung angetrieben wird. Dabei kann beispielsweise gleichzeitig mit der Bewegung in Richtung des Pfeils 3 ein Schwenken des Antriebskörpers um den Angriffspunkt 5 im Uhrzeigersinn um einen bestimmten Winkel, beispielsweise 10°, erfolgen, so dass der Antriebskörper sich ähnlich wie unter der Wirkung eines Fluidgegendrucks zum Ende der Bewegung hin neigt. Optional kann zum Ende der Translationsbewegung die Rotationsrichtung der Schwenkbewegung noch umgekehrt werden, um mit der Flosse zu schlagen. Dieses Antriebsprinzip kann sowohl mit steifen als auch mit flexiblen Antriebskörpern kombiniert werden.

Es kann zu diesem Zweck ein spezieller Hebelantrieb oder ein Kulissenantrieb des Antriebskörpers vorgesehen sein, oder es ist denkbar, mittels einer hydraulischen oder pneumatischen Vorrichtung die Antriebskräfte zu übertragen.

Fig. 2 zeigt in einer Seitenansicht ein Gehäuse 10, in dem eine erfindungsgemäße Fördereinrichtung mit zwei Antriebskörpern 1, 11 angeordnet ist. Das Gehäuse 10 ist rotationssymmetrisch oder im Querschnitt elliptisch um die Antriebskörper 1, 11 herum aufgebaut und weist einen Zulaufkanal 12 sowie einen Ablaufkanal 13 auf. Durch den Ablaufkanal 13 hindurch ragt eine Fluidleitung 14, die Teil des Antriebssystems ist und die mit einem Antriebsbalg 15 verbunden ist. Über eine nicht dargestellte Drucksteuereinrichtung kann über die Fluidleitung 14 der Antriebsbalg 15 mit einem Über- oder Unterdruck verbunden werden, so dass dieser durch Einströmen eines Fluids oder Abziehen des Fluids aufgepumpt bzw. geschrumpft werden kann.

An den beiden Enden 15a, 15b des Antriebsbalgs 15 ist je ein Antriebskörper 1, 11 befestigt, der durch die Volumenänderungen des Antriebsbalges eine Antriebsbewegung in Richtung der Pfeile 3, 4 durchläuft. Durch eine entsprechende elastische Ausgestaltung des Antriebsbalges 15 oder durch zusätzliche Hebel, die den Balg mit den Antriebskörpern 1, 11 oder die Antriebskörper mit einem festen Punkt des Gehäuses 10 verbinden, kann die translatorische Antriebsbewegung des Antriebsbalges 15 in eine komplexere Bewegungsbahn der Antriebskörper 1, 11 übersetzt werden, die einer Überlagerung der translatorischen Bewegung mit einer Schwenkbewegung entsprechen kann.

Es kann jedoch auch vorgesehen sein, dass die Bewegung der Antriebskörper 1, 11 im Wesentlichen translatorisch erfolgt und diese elastisch ausgebildet sind, um die anhand der Fig. 1 dargestellte elastisch flossenartige Gesamtbewegung auszuführen.

Wenn über die Steuerung des Fluiddrucks in der Fluidleitung 14 der Druck im Antriebsbalg 15 periodisch geändert wird, beispielsweise mehrmals pro Sekunde, so wird dies in eine oszillatorische Bewegung der Antriebskörper 1, 11 übersetzt. Dies resultiert in einer Beschleunigung des in dem Gehäuse 10 befindlichen Fluids in Richtung des Pfeils 16, der die Förderrichtung des Fluids bezeichnet. Da durch die Periodizität der Bewegung Druckschwankungen auftreten werden, kann es sinnvoll sein, ein Rückschlagventil 17 im Einlaufkanal 12 vorzusehen, das für den Fall, dass vor dem Ventil innerhalb des Gehäuses 10 ein Überdruck entsteht, den Einlaufkanal 12 sperrt und diesen wieder öffnet, sobald dort ein Unterdruck erzeugt ist.

Die Fluidleitung 14 kann als flexible Schlauchleitung ausgeführt werden, sofern der Antriebsbalg 15 anderweitig in dem Gehäuse 10 gehalten ist. Die Antriebsleitung 14 kann jedoch auch als starre Leitung in Form eines Rohres ausgebildet sein, um gleichzeitig das Fluid zu leiten und den Antriebsbalg sowie die Antriebskörper 1, 11 zu fixieren. In jedem Fall kann die Fluidleitung 14 in einem Haltestern 18 oder an einem Haltearm innerhalb des Ablaufkanals 13 gehalten und fixiert sein.

In der Figur sind für jeden Antriebskörper 1, 11 drei Positionen gezeigt, wobei eine mittlere neutrale Position mit durchgezogenen Linien dargestellt ist und die Extrempositionen auf der Bewegungsbahn jedes einzelnen Antriebskörpers 1, 11 gestrichelt dargestellt sind.

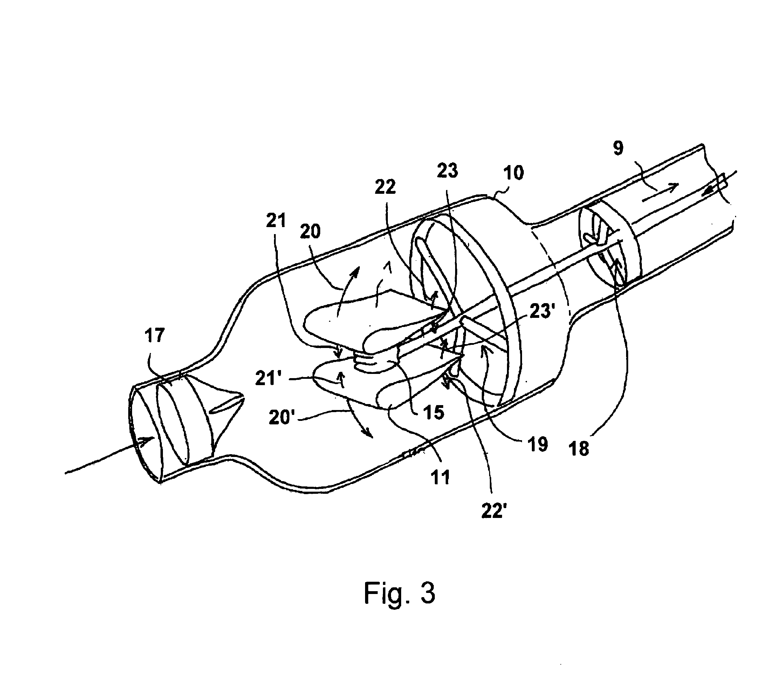

Die Fig. 3 zeigt eine ähnliche Anordnung wie Fig. 2, jedoch in dreidimensionaler Ansicht, wobei ein zweiter Haltestern 19 zusätzlich zu dem ersten Haltestern 18 in unmittelbarer Nachbarschaft des Antriebsbalges 15 und der Antriebskörper 1, 11 vorgesehen ist.

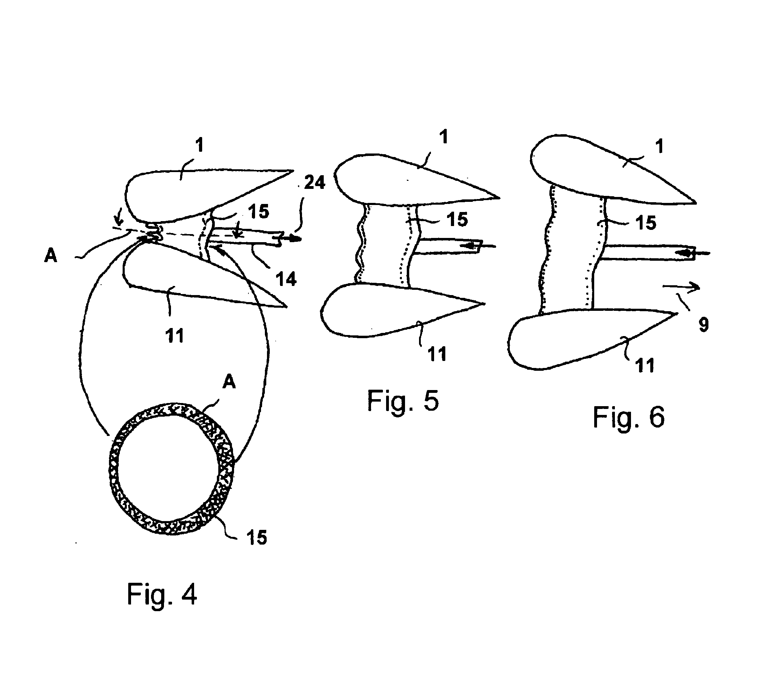

Es sind Pfeile 20, 21 und 20', 21' eingezeichnet, die die Bewegungsrichtungen der jeweils verdickten Enden der Antriebskörper 1, 11 andeuten, sowie Pfeile 22, 23 und 22', 23', die die Bewegung der verjüngten Enden der Antriebskörper 1, 11 andeuten. Die unterschiedliche Länge der dargestellten Pfeile soll andeuten, dass die verdickten, dem Einwegventil 17 zugewandten Enden der Antriebskörper 1, 11 eine Schwenkbewegung ausführen, deren Amplitude wesentlich größer ist als die Bewegung der verjüngten Enden der Antriebskörper. Dies wird, wie näher anhand der Fign. 4, 5 und 6 gezeigt wird, durch eine besondere Konstruktion des Antriebsbalges 15 ermöglicht.

Die Fig. 4 zeigt in einer Seitenansicht im oberen Teil die beiden Antriebskörper 1, 11 sowie den Antriebsbalg 15 in geschrumpfter, d.h. komprimierter Form. Der Pfeil 24 deutet an, dass in diesem Zustand ein Unterdruck in der Fluidleitung 14 besteht, um den Antriebsbalg 15 zu komprimieren.

Der Antriebsbalg 15 selbst ist asymmetrisch aufgebaut, wie aus dem unteren Teil der Fig. 4 genauer hervorgeht. Dort ist ein Querschnitt durch den Antriebsbalg 15 entlang der gestrichelten Linie A gezeigt, der ersichtlich macht, dass der Antriebsbalg in seinem dem Einwegventil 17 zugewandten Bereich eine geringere Wandstärke aufweist als in dem dem Abflusskanal 13 zugewandten Bereich.

Dadurch wird erreicht, dass die Bewegungsamplitude im vorderen, dem Zuflusskanal 12 zugewandten Bereich größer ist als im hinteren, dem Abflusskanal 13 zugewandten Bereich des Antriebsbalges. Dadurch ergibt sich eine Schwenkbewegung der Antriebskörper 1, 11 bei einer Druckänderung in dem Antriebsbalg 15.

In der Fig. 5 ist die Anordnung aus der Fig. 4 mit den Antriebskörpern 1, 11 und einem gegenüber der Fig. 4 weiter aufgepumpten Antriebsbalg 15 gezeigt. Die Antriebskörper befinden sich etwa in der in Fig. 2 dargestellten geraden Position.

Die Fig. 6 letztlich zeigt den Zustand der Antriebskörper 1, 11 im voll aufgepumpten Zustand des Antriebsbalges 15, wobei auch deutlich wird, dass die verdickten Enden der Antriebskörper 1, 11 eine größere Bewegungsamplitude durchlaufen haben als die verjüngten Enden, so dass zusätzlich zu einer translatorischen Bewegung eine Schwenkbewegung der Antriebskörper erfolgt ist.

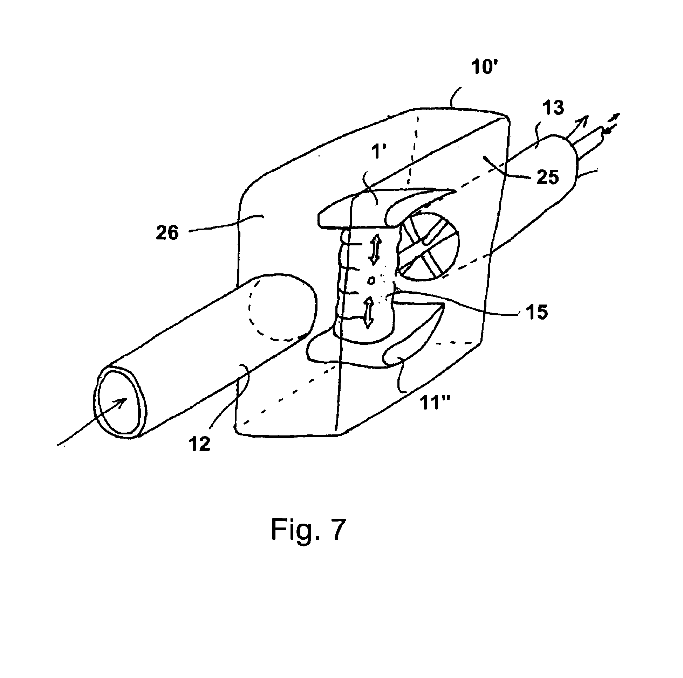

Fig. 7 zeigt aus einer anderen Perspektive dreidimensional dargestellt zwei Antriebskörper 1', 11', die asymmetrisch nach Art eines aerodynamischen Tragflächenprofils ausgebildet sind, jedoch zusätzlich gegebenenfalls flexibel ausgeführt sein können und die mittels eines Antriebsbalgs 15 antreibbar sind. Im Vordergrund der Figur ist der Einlaufkanal 12, im Hintergrund der Auslaufkanal 13 dargestellt. Im Gegensatz zu dem in Fig. 3 dargestellten zylindrischen Gehäuse 10 der Anordnung ist das in der Fig. 7 dargestellte Gehäuse 10' quaderförmig mit rechteckigem Querschnitt aufgebaut, um den nicht zylindersymmetrischen Aufbau der Antriebsanordnung und der Antriebskörper möglichst effizient umzusetzen. Im Unterschied zu der konkreten Darstellung der Fig. 7 kann der Übergang von dem Gehäuse 10' zu den Einlauf-und Auslaufkanälen 12, 13 mit konischen oder schrägen Übergängen erfolgen. Es kann vorteilhaft vorgesehen sein, dass sich die Antriebskörper 1', 11' senkrecht zur Ebene der Antriebsbewegung bis möglichst nah an die Seitenwände 25, 26 des Gehäuses 10' erstrecken. Dadurch werden Verwirbelungen an den Seitenflächen der Antriebskörper 1', 11' reduziert.

Die Antriebskörper 1', 11' können ebenso wie die weiter oben dargestellten Antriebskörper 1, 11 aus einem Schaumstoff, insbesondere Polyurethan, bestehen und aufpumpbar sein. Dazu können die Körper große und/oder viele kleine Hohlräume aufweisen, die beispielsweise durch das Antriebsfluid über die Fluidleitung 14 aufgepumpt werden können und die über Rückschlagventile verfügen, um im aufgepumpten Zustand stabilisiert zu sein.

Hierdurch wird eine gute Komprimierbarkeit im nicht aufgepumpten Zustand ermöglicht, so dass die Antriebskörper zum Transport an einen Einsatzort gemeinsam mit dem Gehäuse 10, 10' radial komprimiert und vor Ort expandiert werden können, bevor sie in Betrieb genommen werden.

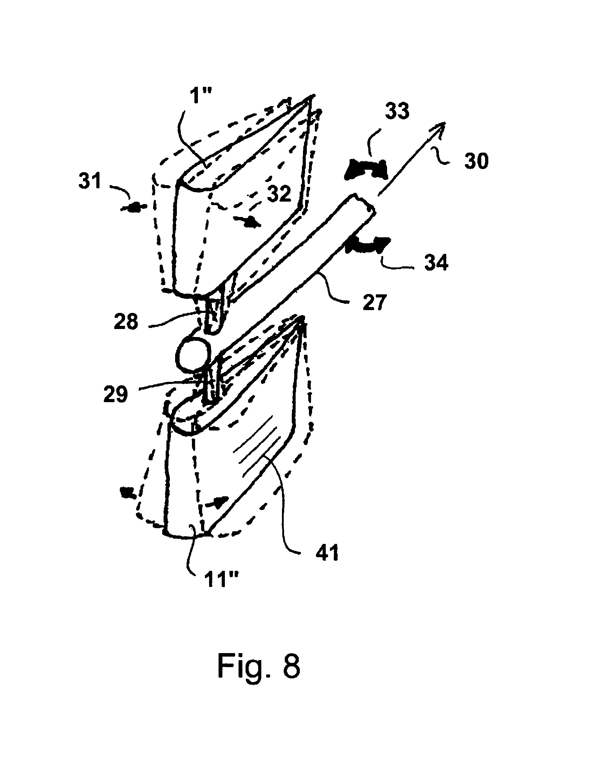

Fig. 8 zeigt im Vergleich zu den weiter oben beschriebenen Figuren eine Anordnung mit zwei Antriebskörpern 1 " , 11 " mit einem anderen Antriebsprinzip, bei dem die Antriebskörper über Verbindungsstege 28, 29 mit einer Antriebswelle 27 verbunden sind, welche in der Förderrichtung 30 verläuft.

Die Antriebswelle 27 kann um die Förderrichtung 30 herum oszillierend gedreht werden, und zwar jeweils beispielsweise mindestens um einen Betrag von 5°, 10° oder mindestens um 20° oder 30° in jede Richtung, wie durch die Pfeile 33, 34 angedeutet.

Die Antriebskörper 1" und 11" sind mit ihren Längsachsen parallel zur Welle ausgerichtet und durchlaufen im Rahmen dieser rotatorischen Bewegung, sofern die Länge der Verbindungsstege 28, 29 ausreichend ist, eine quasitranslatorische Bewegung in Umfangsrichtung der Welle in den Richtungen, die mit den Pfeilen 31, 32 angedeutet sind. Auf diese Weise lässt sich mittels der Antriebswelle 27 auf sehr einfache Weise eine entsprechende annähernd lineare translatorische Bewegung der Antriebskörper realisieren. In Figur 8 sind beispielhaft am unteren Antriebskörper 11" auch eine Mehrzahl von parallelen Mikrorillen 41 dargestellt.

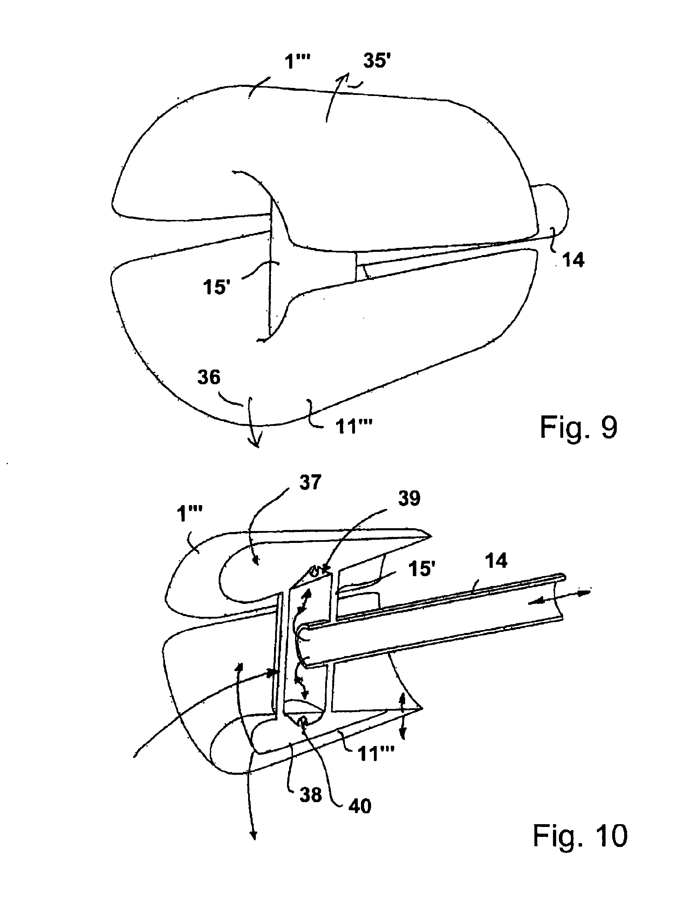

In der Fig. 9 ist in dreidimensionaler Ansicht eine möglichst weitgehend zylindersymmetrische Anordnung von zwei Antriebskörpern 1" ' und 11" ' dargestellt, die mit einem Antriebsbalg 15' verbunden sind und im Wesentlichen in Richtung der Pfeile 35, 36 in radialer Richtung bezüglich der Zylinderachse bewegt werden können. Der Antriebsbalg 15' ist mittels einer Fluidleitung 14 mit einem Druckerzeugungssystem verbunden. Es ist auch denkbar, die Zylindersymmetrische Anordnung in eine höhere Anzahl von beispielsweise 4 oder 8 oder mehr Zylindersegmenten zu unterteilen und diese jeweils radial zu bewegen, wobei sich ein Bewegungsmuster ergibt, das der Fortbewegungsart von Quallen ähnelt.

In Fig. 10 ist ein Schnitt durch die Anordnung aus Fig. 9 dargestellt, der die Funktion deutlich macht. Der Antriebskörper 1 " ' ist beispielhaft mit einem Hohlraum 37 dargestellt, der Antriebskörper 11 " ' mit einem Hohlraum 38, wobei die Hohlräume nur schematisch angedeutet sind.

Über die Fluidleitung 14 wird Fluid mit dem Inneren des Antriebsbalgs 15 ausgetauscht und von dort in die Hohlräume 37, 38 hineingepumpt, wobei die Hohlräume 37, 38 der Antriebskörper 1"' und 11"' mittels Einwegventilen 39, 40 mit dem Hohlraum des Antriebsbalgs 15' verbunden sind, so dass die Antriebskörper nur einmal aufgepumpt werden und danach den erhöhten Fluiddruck halten, um sich in der Form zu stabilisieren. Danach wird lediglich das Innere des Antriebsbalgs 15' aufgepumpt und geschrumpft. Dadurch bewegen sich die Antriebskörper 1"', 11"' abwechselnd in Richtung der Pfeile 35, 36 auseinander und in entgegengesetzter Richtung zusammen, wodurch eine entsprechende Antriebsbewegung realisiert wird.

Durch die zylindersymmetrische oder annähernd zylindersymmetrische Anordnung der Antriebskörper wird der Wirkungsgrad der Fördereinrichtung gegenüber den nicht zylindersymmetrischen Anordnungen, die in den oben genannten Figuren dargestellt sind, erhöht.

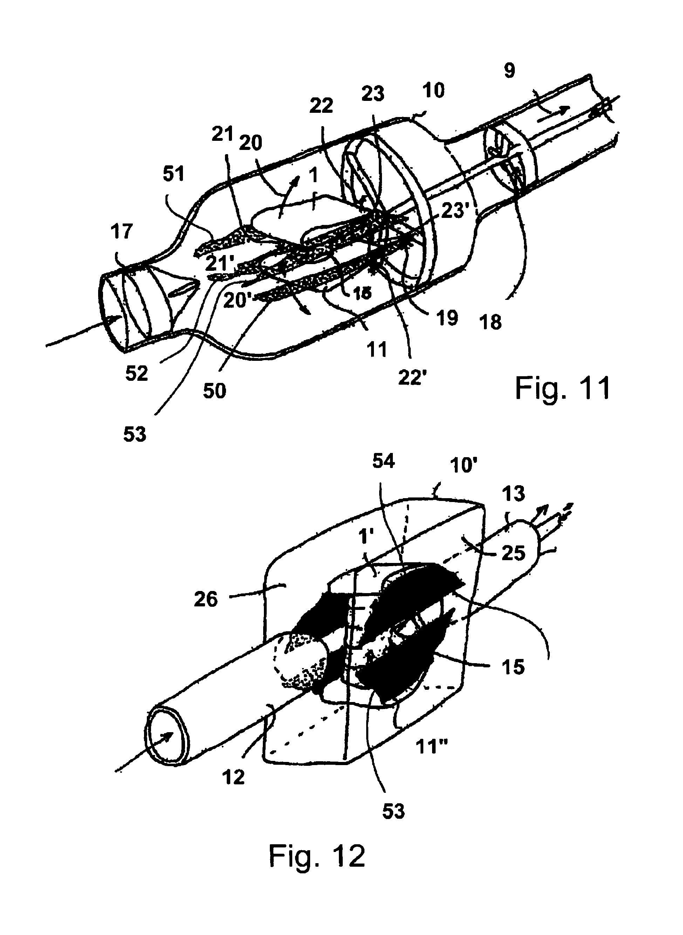

Fig. 11 zeigt eine zylindrische Anordnung eines Gehäuses 10 mit zwei Antriebskörpern 11, die seitlich jeweils mit Sperrkörpern 50, 51, 52, 52 versehen sind, die flexibel sind und auch mit der Wand des Gehäuses 10 verbunden sein können und die während der Antriebsbewegung einen Druckausgleich zwischen Unter-und Oberseite bzw. der Hochdruck- und Niederdruckseite jedes Antriebskörpers verhindern oder verringern.

Fig. 12 zeigt entsprechende Sperrkörper 53, 54 für ein Gehäuse 10' mit abgeflachten Seitenwänden.

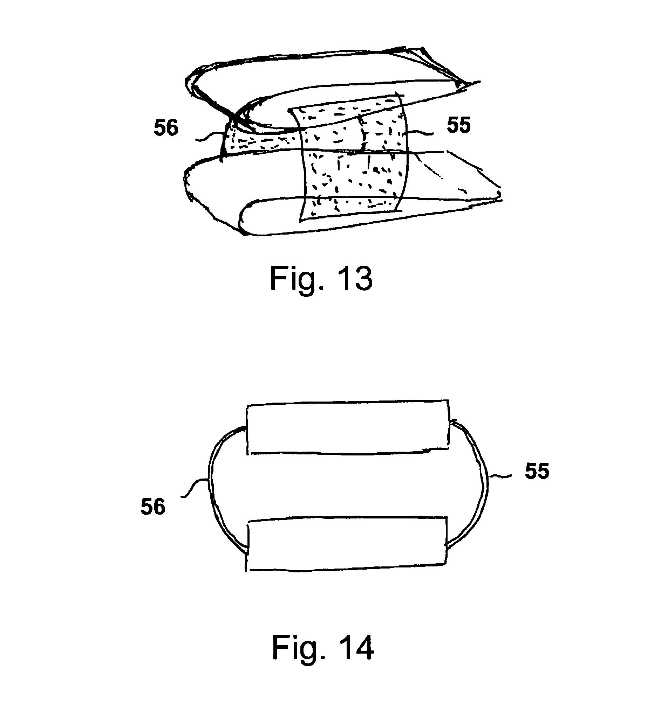

Fig. 13 zeigt zwei Sperrkörper in Form von breiten, flexiblen Bändern 55, 56, die zwei Antriebskörper zu beiden Seiten miteinander verbinden. Diese Konstellation ist in Fig. 14 in einer Frontansicht gezeigt.

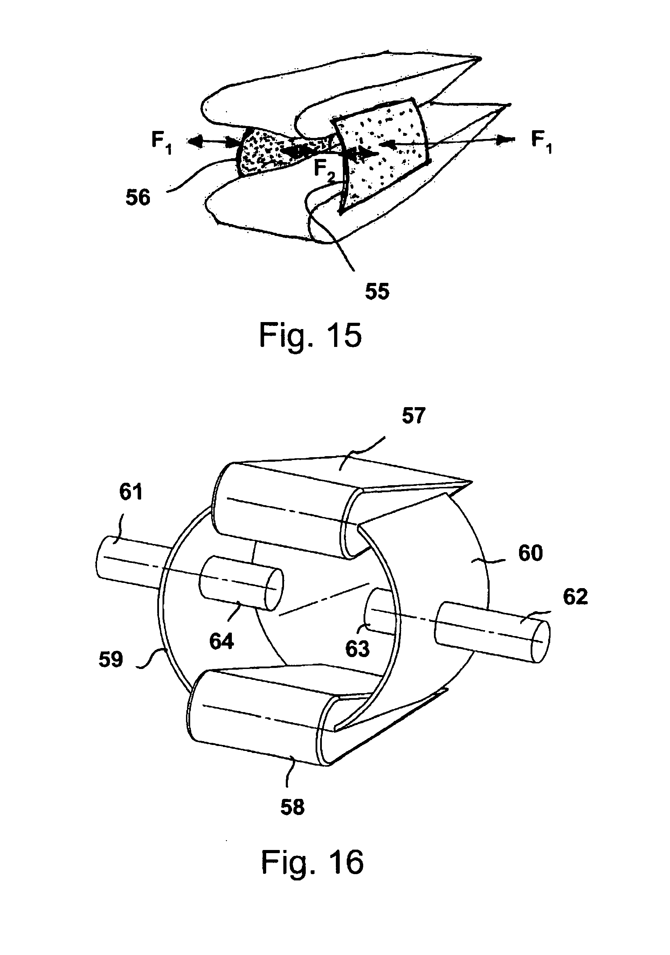

Fig. 15 zeigt zwei Sperrkörper 55, 56 wie in Fig. 13, die zwei flossenförmige Antriebskörper miteinander verbinden und als Ausgleichssperre wirken. Die Sperrkörper sind als Streifen ausgebildet und können biegeschlaff oder steif und elastisch biegbar ausgebildet sein. Im letzten Fall kann durch gezieltes Aufbringen einer mechanisch, magnetisch, pneumatisch, hydraulisch oder elektrisch erzeugten Antriebskraft auf die Sperrkörper von außen, angedeutet durch die Pfeile F₁ und F₁', oder von innen aus dem Zwischenraum der Antriebskörper, angedeutet durch den Doppelpfeil F₂, eine Antriebsbewegung auf die Antriebskörper gezielt aufgebracht werden.

Anstelle der Sperrkörper können auch ähnlich positionierte Kopplungskörper in Form eines Gerüstes oder Rahmens zur Einkopplung der Antriebsbewegung in die Profile vorgesehen sein.

Das Prinzip des Antriebs über die Sperrkörper ist beispielhaft zusätzlich durch Fig. 16 verdeutlicht. Dort sind zwei Antriebskörper 57, 58 durch zwei Ringsegmente 59, 60 eines Ringstreifens in Form eines Kreisrings miteinander verbunden. Die Zylinder 61, 62 deuten symbolisch außen ansetzende Antriebskräfte an, die von außen auf die Ringsegmente eine Zug- oder Druckkraft aufbringen können. Mit 63, 64 sind symbolisch entsprechende, innen ansetzende Kräfte bezeichnet. Eine Verformung der Ringsegmente bewirkt eine Antriebsbewegung der Antriebskörper 57, 58. Diese kann in geeigneter Weise durch eine Profilierung der Ringsegmente 59, 60 oder durch Ausnehmungen in den Ringsegmenten gesteuert werden.

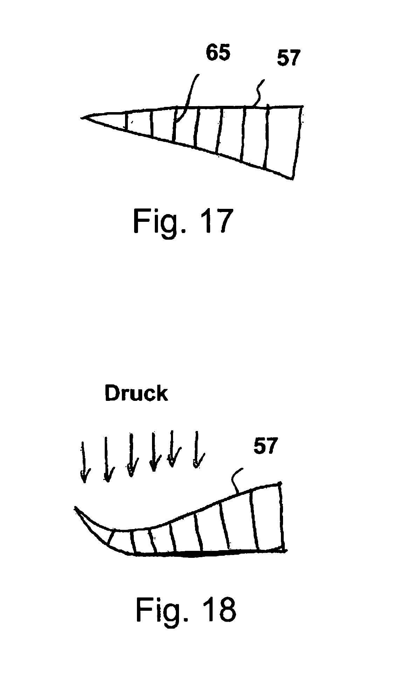

In Fig. 17 und 18 ist in einer schematischen Draufsicht ein Antriebskörper mit sogenannten fin-rays 65 gezeigt, die als steg-, nut- oder flossenähnliche Strukturen auf der Oberfläche einen Einfluss auf die Strömung des Fluids nehmen. Diese können derart geformt und gestaltet sein, dass sie bei Bewegung des Antriebskörpers auf der Druckseite eine konkave Verformung und damit eine Druckerhöhung bewirken.

Die erfindungsgemäße Fördereinrichtung für Fluide erlaubt durch den Einsatz einer oszillatorischen Bewegung quer zur Förderrichtung von Antriebskörpern eine effiziente Ausgestaltung, wobei die Nachteile von ausschließlich rotierenden Antriebseinrichtungen vermieden werden.

## Patentansprüche

1. Fördereinrichtung zur Förderung eines Fluids in einer Förderrichtung mit wenigstens einem mittels eines Antriebssystems antreibbaren Antriebskörper (1,1',1",1"',11,11',11",11"'), **dadurch gekennzeichnet, dass** der Antriebskörper quer zur Förderrichtung (9) oszillierend antreibbar ist.

2. Fördereinrichtung nach Anspruch 1, **dadurch gekennzeichnet, dass** wenigstens eine Förderfläche (6,7) wenigstens eines Antriebskörpers (1,1',1",1"',11,11',11",11"') derart ausgerichtet ist, dass bei Bewegung des Antriebskörpers auf das Fluid eine Teilkraft in der Förderrichtung (9) wirkt.

3. Fördereinrichtung nach Anspruch 2, **dadurch gekennzeichnet, dass** zwei Förderflächen (6,7) derart ausgerichtet sind, dass sie in jeweils wenigstens einer Bewegungsrichtung (3,4) eines Antriebskörpers (1,1',1",1"',11,11',11",11"') eine Förderung des Fluids bewirken.

4. Fördereinrichtung nach Anspruch 1, 2 oder 3,
**dadurch gekennzeichnet, dass** wenigstens ein Antriebskörper (1,1',1'',1''',11,11',11'',11''') sich in Förderrichtung (9) in dem parallel zu seiner Bewegungsebene liegenden Querschnitt verjüngt.

5. Fördereinrichtung nach Anspruch 1 oder einem der folgenden, **dadurch gekennzeichnet, dass** der wenigstens eine Antriebskörper (1,1',1",1"',11,11',11",11"') steif ausgebildet ist.

6. Fördereinrichtung nach einem der Ansprüche 1 bis 4, **dadurch gekennzeichnet, dass** der Antriebskörper (1,1',1'',1''',11,11',11'',11''') derart elastisch ausgebildet ist, dass er durch den Fluidgegendruck im Betrieb in seinem Endbereich um wenigstens 5° gegenüber dem unverformten Zustand biegbar ist.

7. Fördereinrichtung nach einem der Ansprüche 1 bis 6, **dadurch gekennzeichnet, dass** der Antriebskörper in Förderrichtung (9) verlaufende Mikrorillen (41) aufweist.

8. Fördereinrichtung nach Anspruch 1 oder einem der folgenden, **dadurch gekennzeichnet, dass** der Antriebskörper (1,1',1",1"',11,11',11",11"') wenigstens einen Hohlraum (37,38) aufweist.

9. Fördereinrichtung nach Anspruch 8, **dadurch gekennzeichnet, dass** der Antriebskörper (1,1',1'',1''',11,11',11'',11''') aus einem Schaumstoff, insbesondere Polyurethan, besteht.

10. Fördereinrichtung nach Anspruch 8 oder 9, **dadurch gekennzeichnet, dass** der Antriebskörper wenigstens (1,1',1'',1''',11,11',11'',11''') teilweise aufpumpbar ist.

11. Fördereinrichtung nach Anspruch 1 oder einem der folgenden, **dadurch gekennzeichnet, dass** der Antriebskörper mittels einer hydraulischen oder pneumatischen Einrichtung (15,15'), insbesondere eines Ballonkörpers, antreibbar ist.

12. Fördereinrichtung nach einem der Ansprüche 1 bis 10, **dadurch gekennzeichnet, dass** der Antriebskörper mittels einer elektrischen und/oder magnetischen Einrichtung antreibbar ist.

13. Fördereinrichtung nach Anspruch 1 oder einem der folgenden, **dadurch gekennzeichnet, dass** wenigstens ein Antriebskörper (1,1',1",1"',11,11',11",11"') oszillierend um eine quer zur Förderrichtung (9) verlaufende Achse schwenkbar ist.

14. Fördereinrichtung nach einem der Ansprüche 1 bis 13, **dadurch gekennzeichnet, dass** der/die Antriebskörper (1", 11'') oszillierend um eine in Förderrichtung (9), insbesondere außerhalb des/der Antriebskörper(s) verlaufende Achse schwenkbar sind.

15. Fördereinrichtung nach einem der Ansprüche 1 bis 14, **gekennzeichnet durch** seitlich an wenigstens einem Antriebskörper befestigten Sperren, die eine Barriere zwischen verschiedenen Förderflächen eines Antriebskörpers bilden.

16. Fördereinrichtung nach Anspruch 14, **dadurch gekennzeichnet, dass** wenigstens eine Sperre entweder mit zwei Antriebskörpern oder mit einem Antriebskörper und einem Gehäuse der Fördereinrichtung verbunden ist.

17. Fördereinrichtung nach Anspruch 15 oder 16, **dadurch gekennzeichnet, dass** die Antriebskraft auf die Antriebskörper mittels des/der Sperrkörper(s) aufgebracht wird.
